# DEMANDE DE BREVET EUROPEEN

(11) **EP 2 692 316 A1**
(43) Date de publication de la demande: **05.02.2014**
(21) Numéro de dépôt: 12178981.2
(22) Date de dépôt: 02.08.2012
(51) Int. Cl.: A61F 3/00

(54) **Dispositif de rééducation pour jambes de longueurs inégales.**

(71) Demandeur: Paraplan Sprl, 1170 Bruxelles (BE)
(72) Inventeur: Dandoy, Gilles, 1640 Rhode St-Genèse (BE)
(74) Mandataire: Decamps, Alain René François

(57) **Abrégé**

Dispositif pour le traitement de jambes de longueurs inégales comprenant un patin (4) apte à être fixé à une chaussure (10). Le patin (4) est une bande rigide en substance plate repliée en forme de C couché. Il comprend une partie centrale cintrée et deux extrémités rentrantes, raccordées par des premiers moyens de fixation (8) à une semelle plane(2). Cette semelle (2) est apte à être fixée de façon semi-permanente à l'aide de seconds moyens de fixation (12) à la semelle d'une chaussure (10).

## Description

### Domaine de l'invention

L'invention se rapporte à un dispositif (orthèse) pour traiter les pathologies impliquant des jambes de longueurs inégales (en anglais LLI pour « leg length inequality » ou LLAA pour « leg length alignment asymmetry »).

Ce dispositif est adapté à un usage thérapeutique, rééducatif, mais également de confort.

### Préambule

Des études ont montré que la plupart des êtres humains (on a évoqué une proportion de l'ordre de 90 %) présentent une différence de longueur moyenne de l'ordre de 5 mm entre les deux jambes, sans s'en être jamais rendu compte. Les muscles et les articulations compensent en effet sans trop de problème cette différence, tant qu'elle reste minime.

A partir d'un certain seuil, toutefois (typiquement 20 mm), cette différence ne peut plus être compensée « normalement », et entraîne une claudication, voire un déhanchement significatif pour l'oeil des professionnels.

Les causes de cette différence de longueur sont variées : elles peuvent être congénitales ou apparaître suite à un traumatisme (accident, fracture, placement d'une hanche artificielle, dégénérescence de l'os, etc.).

Outre des difficultés liées à la marche, cette pathologie a pour conséquence des problèmes notamment au niveau des genoux et de la hanche.

On remarquera que la LLI, qui affecte les os des jambes (fémur, tibia et péroné), n'a rien de commun avec le « pied bot », qui est en fait une déformation des os et des muscles du pied.

### État de la technique

Le seul traitement courant pour la LLI est le placement d'une talonnette ou l'usage de chaussures orthopédiques à semelles épaisses réalisées sur mesure.

Ce type de chaussures se caractérise par une semelle hyper-compensée, du type précisément de ce qui est utilisé pour les pieds bots.

Ces chaussures, confectionnées par des spécialistes prothésistes, sont très onéreuses et il n'est évidemment pas possible d'en changer régulièrement pour suivre la mode, par exemple.

Outre leur aspect peu esthétique, ces chaussures visent à assurer une stabilité maximale à leur porteur, et en conséquence, elles sont munies d'une semelle plate, ce qui réduit le degré de liberté des articulations au niveau du genou et de la hanche et accentue encore les problèmes de claudication.

US-3 924 615, considéré comme le plus proche état de la technique, décrit une semelle de compensation pour pied-bot. Cette semelle, formée d'une plaque pliée en U, est destinée à être fixée à l'aide de lanières à une chaussure. Comme mentionné ci-dessus, la surface en contact avec le sol est plane et ne facilite en rien la marche.

On connaît par KR-20100005414 U ou CN-2321260 U, CN-102302241 des chaussures à semelles à ressort, développées pour le jeu, le jogging ou la pratique de sports extrêmes. Leur propos est d'emmagasiner l'énergie cinétique développée par le coureur pour le faire rebondir, lui permettant d'accomplir de grandes enjambées. Ce type de chaussures est à l'évidence totalement inadapté pour le traitement de la LLI.

Enfin, la mode et le design mettent régulièrement sur le marché de semelles répondant à des postulats divers (talon surbaissé, etc.) sur le confort du pied, tels que par exemple KR-20090003909.

### Résumé de l'invention

Un but de l'invention est de faciliter le port d'une orthèse pour les personnes souffrant de LLI.

Un autre but de l'invention est de favoriser la rééducation des muscles de la jambe, de façon à réduire ou, à terme, d'éliminer la claudication.

L'objet de l'invention est un dispositif pour le traitement de jambes de longueurs inégales comprenant un patin apte à être fixé à une chaussure, ce patin étant une bande rigide en substance plate repliée en forme de C couché. Ce patin comprend une partie centrale cintrée et deux extrémités rentrantes, raccordées à une semelle plane. Cette semelle est apte à être fixée de façon semi-permanente à l'aide de seconds moyens de fixation à la semelle d'une chaussure.

De façon avantageuse, les seconds moyens de fixation sont des vis, la tête de ces vis affleurant, après serrage, une face supérieure de la semelle de la chaussure.

Un avantage de l'invention est que l'on peut l'adapter, suivant son goût, sur des chaussures ordinaires, ce qui le rend beaucoup plus discret qu'une chaussure orthopédique classique.

La distance entre la semelle et le patin est de préférence réglable. Le dispositif peut notamment comprendre une cale d'épaisseur apte à être insérée entre le patin et la semelle ; suivant une forme de réalisation optionnelle, des premiers moyens de fixation raccordant les deux extrémités rentrantes et la semelle plane comprennent une tige filetée permettant de régler la distance entre la semelle et le patin ; suivant une autre forme de réalisation optionnelle, le dispositif comprend des moyens permettant d'agir sur la forme du patin.

De la sorte, le patient peut régler lui-même finement la position du patin, sans passer forcément par l'orthopédiste.

Le patin est avantageusement réalisé en un matériau métallique, choisi par exemple parmi l'aluminium, le titane est leurs alliages.

Suivant une autre forme de réalisation avantageuse, le patin est réalisé en un matériau plastique.

La surface inférieure du patin est de préférence recouverte au moins partiellement par un matériau élastique. Ce matériau élastique est avantageusement antidérapant.

Suivant une forme de réalisation préférée, le patin présente une extension vers l'extérieur du pied de façon à prévenir une éventuelle torsion de la cheville lors de la marche.

Un autre objet de l'invention est un procédé de fabrication d'un dispositif tel que décrit plus haut qui comprend les opérations suivantes :
- Disposer d'un ordinateur ;
- Implémenter dans cet ordinateur un programme de simulation de la marche humaine dans lequel les dimensions anthropométriques d'un individu fictif affecté de LLI sont utilisées comme variables ;
- Implémenter dans ce programme la possibilité d'intégrer en outre la présence d'un dispositif de correction doté d'un patin, monté sur une des jambes ;
- Stocker dans un sous-programme des formes et des dimensions variables pour le patin de ce dispositif ;
- Prendre les mensurations anthropométriques d'un patient ;
- Entrer ces mensurations comme variables dans le programme de simulation de l'ordinateur ;
- Ajouter à ces variables la présence d'un dispositif de correction avec patin de forme arbitraire ;
- Vérifier la correction de la marche ainsi obtenue ;
- Faire varier la forme et les dimensions du patin ;
- Déterminer formes et dimensions du patin procurant la correction optimale ;
- Confectionner un patin correspondant à cette forme optimale.

Suivant une forme de réalisation préférée, le procédé comprend en outre l'opération suivante :
- Appliquer sur au moins une partie de la face inférieure du patin une couche de matériau élastique.

Outre ses propriétés anti-dérapantes, cette couche élastique absorbera les imperfections du sol: gravillons, revêtements pavés, etc. On peut utiliser, par exemple, une tranche de mousse d'élasticité adéquate.

### Brève description des figures

Ces aspects ainsi que d'autres aspects de l'invention seront clarifiés dans la description détaillée de modes de réalisation particuliers de l'invention, référence étant faite aux dessins des figures, dans lesquelles :
- La Fig.1: est une vue en perspective d'une forme de réalisation du dispositif de l'invention ;
- La Fig.2: est une vue en perspective d'une autre forme de réalisation du dispositif de l'invention ;
- La Fig.3: est une vue en perspective dispositif de l'invention de la Fig. 2 monté sur une chaussure;
- Les Fig. 4 et 5: sont des vues en plan d'un patin du dispositif de l'invention ;
- La Fig. 6: est une vue du dessus du dispositif de la Fig. 1 ;
- La Fig. 7: est une vue éclatée d'une forme de réalisation du dispositif de la Fig. 1 ;
- Les Fig. 8 à 10: sont des vues de la démarche d'une personne équipée d'un dispositif de l'invention.
- Les Fig. 11 à 13: sont des vues latérales schématiques de différentes positions du dispositif au cours de la marche ;
- Les Fig. 14 et 15: sont des vues latérales schématiques de deux autres formes de réalisation du dispositif de l'invention.

Les figures ne sont pas dessinées à l'échelle. Généralement, des éléments semblables sont dénotés par des références semblables dans les figures.

### Description détaillée de modes de réalisation particuliers

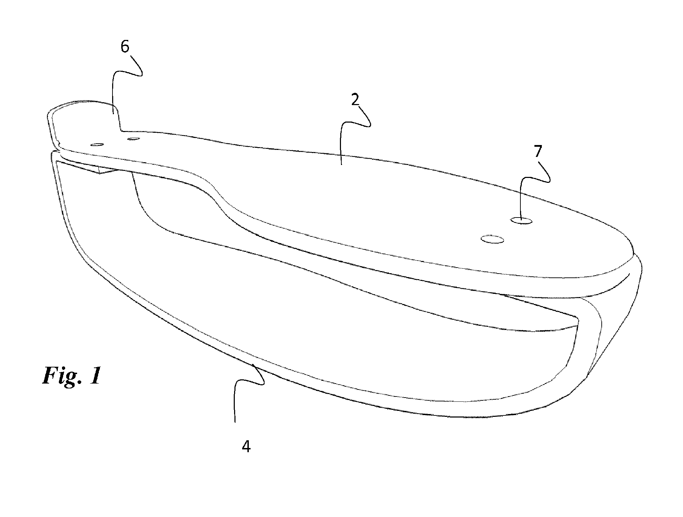

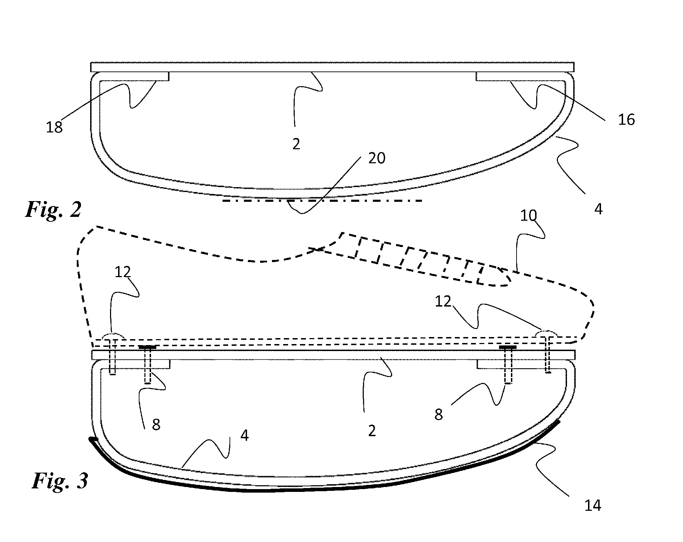

Les Figures 1 et 2 montrent deux formes de réalisation du dispositif de l'invention. Ce dispositif est muni d'une semelle 2, dont la forme correspond sensiblement à celle d'une semelle de chaussure, et d'un patin 4, de forme cintrée. La semelle 2 montrée à la Fig. 1 comprend une butée de talon 6 pour des marches sur terrain difficile. Semelle et patins sont munis de trous traversants 7 destinés à accueillir des premiers moyens de fixation 8, tels que des boulons 8 ou tiges filetées (comme représentés à la Fig. 3).

La Fig. 3 montre le dispositif fixé à une chaussure 10, représentée ici en pointillé. Cette chaussure a ici la forme d'une chaussure de ville, mais on aurait pu choisir toute autre forme de chaussure, au gré de la fantaisie de l'utilisateur. Un des avantages du dispositif de l'invention est, en effet, de pouvoir s'adapter à la tenue de son utilisateur, ce qui le rend particulièrement discret et contribue donc à la réinsertion sociale des personnes affectées de LLI.

On note sur la Fig. 3 la présence de premiers moyens de fixation 8 reliant la semelle 2 et le patin 4, ainsi que de deuxièmes moyens de fixation (boulons) 12, qui traversent la semelle de la chaussure 10. La tête des boulons 12 est évidemment choisie pour ne pas gêner la plante des pieds de l'utilisateur, et peut être surmontée d'une semelle anti-transpiration ou tout autre accessoire du genre.

La légèreté du dispositif est un facteur décisif de confort. En conséquence, la semelle et/ou le patin seront constitués de préférence de métal léger (aluminium, titane, etc.) ou de matériaux plastiques à hautes performances (renforcés par exemple de fibres de verre voire de carbone). On peut en outre envisager l'usage d'autres matériaux présentant des caractéristiques mécaniques adéquates, tels que le bois imprégné, lamellé-collé, etc.

En fonction de la nature du patin et/ou de la semelle, les boulons 8, 10 peuvent être engagés dans des écrous ou dans des trous taraudés. Les critères à appliquer sont évidemment la légèreté de l'ensemble [dispositif + chaussure] et la fiabilité de la fixation. Si l'usager compte faire un usage fréquent de la possibilité de monter le dispositif sur des chaussures de styles variés, l'utilisation de clips permet un démontage-remontage encore plus rapide du dispositif. On peut également envisager un montage temporaire sur des chaussures non-adaptées à l'aide, notamment, de rubans auto-agrippant de type Velcro.

L'usage du mot « patin » ne doit pas faire oublier que le dispositif doit assurer à son utilisateur une démarche aussi « normale » que possible. En conséquence, une couche élastique antidérapante 14 est prévue au moins sous la partie centrale du patin 4, qui porte sur le sol. L'épaisseur de cette couche est généralement d'environ 5mm et il en est tenu compte lors du calcul des dimensions du patin 4.

Comme il apparaît sur les différentes Figures, le patin 4 affecte la forme d'un « C » couché, ses extrémités rentrantes 16, 18 se raccordant à la semelle 2.

La courbure du patin 4 ne doit rien au hasard.

On a en effet constaté que, de façon paradoxale, le port régulier, voire permanent, de chaussures munies du dispositif de l'invention entraîne une rééducation des muscles des genoux et de la hanche (ce que ne permet pas une chaussure orthopédique classique). Le développement de la courbe du patin sur le sol sollicite les muscles atrophiés par le raidissement permanent de la jambe trop courte. Ceux-ci reprennent donc progressivement une certaine élasticité et du tonus, avec un effet positif sur la claudication et le redressement de la colonne vertébrale.

Il est donc important non seulement d'étudier soigneusement cette courbure pour l'adapter à chaque patient, mais encore de l'adapter régulièrement au progrès réalisés.

La position de l'apex (point bas de la courbe) 20, notamment, est déterminante, puisqu'elle fixe l'endroit où le patin touche le sol lors de la marche. Suivant les habitudes et le confort du patient, l'apex peut se trouver au milieu de la courbe, comme représenté à la Fig. 3, davantage vers la pointe du pied (meilleur amortissement des chocs), ou davantage vers l'arrière (jambe trop raide).

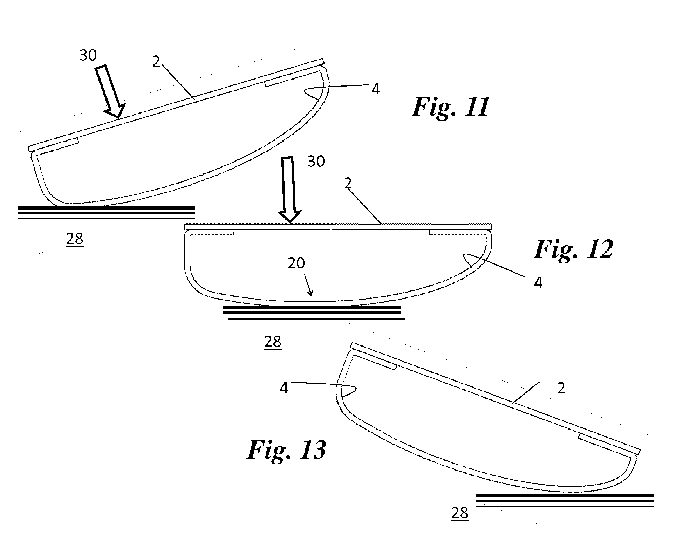

Ce point est développé plus en détail ci-dessous, en référence aux Fig. 11 à 13.

La courbure adéquate du patin 4 et son développé peuvent être réalisés de façon classique, par des essais successifs, à partir de gabarits standardisés ou mieux à l'aide d'un programme informatique déterminant, en fonction des caractéristiques anthropométriques du patient, la forme théorique idéale à différents stades de la rééducation à la marche.

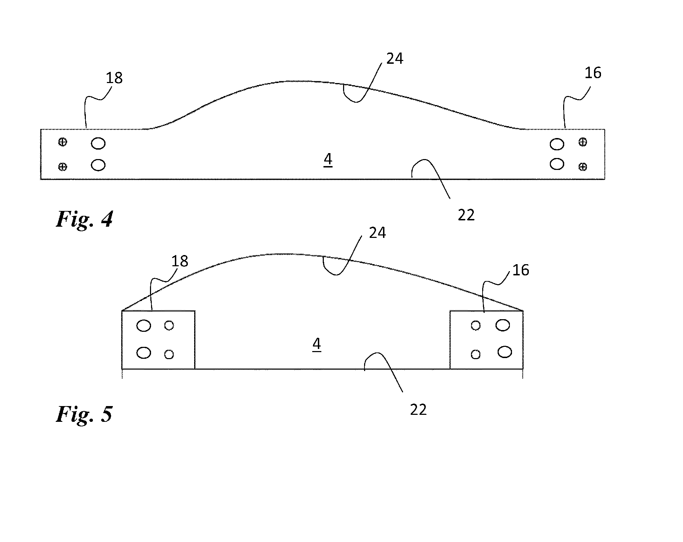

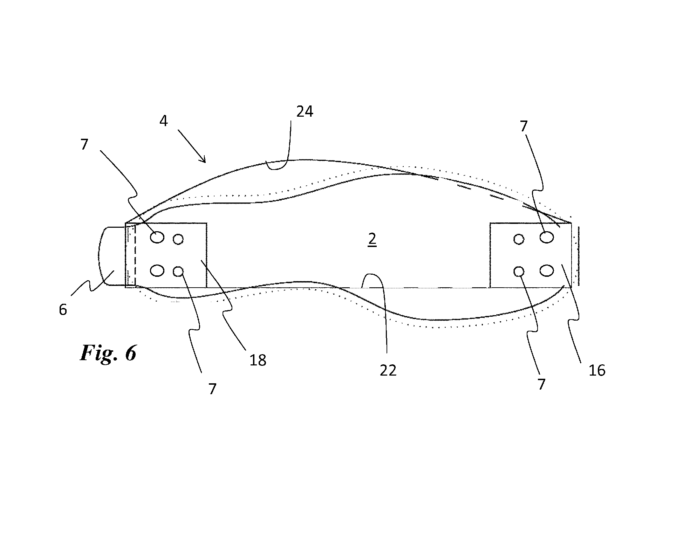

Les Fig. 4, 5 et 6 montrent le patin 4 sous sa forme aplatie, après courbure et après assemblage. On remarque que le bord proximal 22 (dirigé vers la face interne de la jambe) du patin est pratiquement rectiligne (afin de gagner du poids), alors que le bord distal 24 présente un élargissement évitant au pied de se tordre.

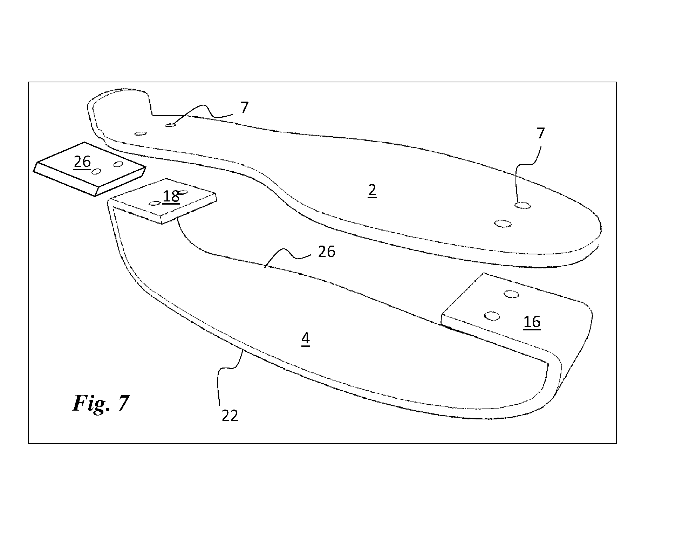

La Fig. 7 est une vue éclatée d'une autre forme de réalisation du dispositif de l'invention, dans laquelle une talonnette (en fait, une cale d'épaisseur 26) est glissée entre la semelle 2 et le patin 4 pour permettre un réglage fin de la hauteur du dispositif.

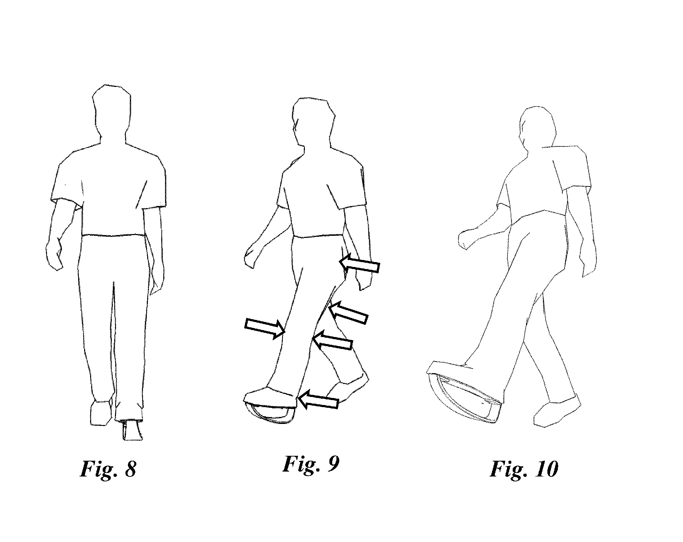

Les Fig. 8 à 10 sont des vues schématiques d'un patient équipé de chaussures dont l'une est munie du dispositif de l'invention, avec indication fléchées des muscles et/ou articulations concernés par la rééducation.

Les Fig. 11, 12, 13 montrent les étapes successives de la marche et l'importance d'une forme adéquate de la courbe du patin.

La vue 11 montre l'instant où le talon touche le sol. La courbure du patin provoque un moment de rotation qui force les muscles du genou à réagir, d'où une rééducation progressive. Si le point de contact avec le sol 28 est trop en arrière, le poids du corps 30 provoque un moment de rotation trop important et le genou aura tendance à se dérober.

A la vue 12, la jambe est en pleine charge et l'apex 20 est censé toucher le sol, de façon à offrir un appui stable pendant que le poids de tout le corps 30 est porté par la jambe affectée. Pendant l'enjambée (Fig. 13), le poids du corps passe progressivement vers la pointe du pied. La hanche est sollicitée, et le genou est amené à se plier légèrement pour ramener la jambe vers l'avant et éviter que le pied traîne sur le sol 28.

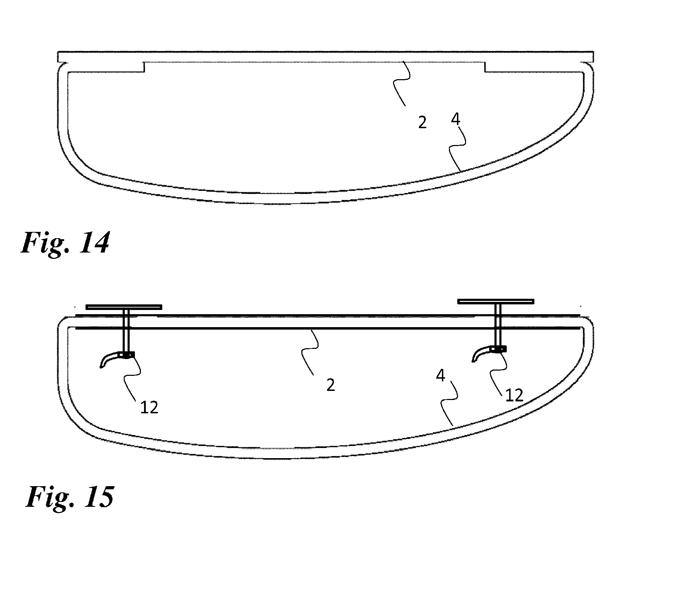

Les Figures 14 et 15 illustrent deux modes de réalisation optionnels du dispositif de l'invention. La Fig. 14 montre un mode de réalisation où le patin 4 et la semelle 2 sont assemblés par soudage (cas de pièces métalliques) ou par collage (cas de pièces plastiques).

A la Fig. 15, semelle 2 et patins 4 sont réalisés d'un seul tenant, par injection ou tout autre procédé similaire. Sur la Fig. 15 apparaissent en outre des seconds moyens de fixation 12 « rapides », en l'occurrence un système à verrouillage muni d'un excentrique actionné par levier. L'utilisateur a ainsi la possibilité d'échanger rapidement de dispositif entre deux paires de chaussures de styles différents (chaussures de ville et chaussures de marche). Les moyens de fixation 12 ici représentés sont équipés d'une barrette transversale propres à mieux fixer la chaussure à la semelle 2, ce qui permet de n'en employer qu'un à l'avant et un à l'arrière de la chaussure.

Il apparaîtra évident pour l'homme du métier que la présente invention, sans s'écarter des revendications ci-après, n'est pas limités aux exemples illustrés et décrits ci-dessus. L'invention comprend chacune des caractéristiques nouvelles ainsi que leur combinaison. La présence de numéros de référence ne peut être considérée comme limitative. L'usage du terme « comprend » ne peut en aucune façon exclure la présence d'autres éléments autres que ceux mentionnés. L'usage de l'article défini « un » pour introduire un élément n'exclut pas la présence d'une pluralité de ces éléments. La présente invention a été décrite en relation avec des modes de réalisations spécifiques, qui ont une valeur purement illustrative et ne doivent pas être considérés comme limitatifs.

## Revendications

1. Dispositif pour le traitement de jambes de longueurs inégales comprenant un patin (4) apte à être fixé à une chaussure (10), **caractérisé en ce que** le patin (4) est une bande rigide en substance plate repliée en forme de C couché, ce patin (4) comprenant une partie centrale cintrée et deux extrémités rentrantes, raccordées à une semelle plane(2), la dite semelle (2) étant apte à être fixée de façon semi-permanente à l'aide de seconds moyens de fixation (12) à la semelle d'une chaussure (10).

2. Dispositif selon la revendication 1, **caractérisé en ce que** les seconds moyens de fixation sont des vis, la tête de ces vis affleurant, après serrage, une face supérieure de la semelle de la chaussure (10).

3. Dispositif selon l'une quelconque des revendications 1 ou 2, **caractérisé en ce que** la distance entre la semelle et le patin est réglable.

4. Dispositif selon la revendication 3, **caractérisé en ce que** qu'il comprend une cale d'épaisseur (26) apte à être insérée entre le patin (4) et la semelle (2) du dispositif.

5. Dispositif selon l'une quelconque des revendications 3 ou 4, **caractérisé en ce que** des premiers moyens de fixation (8) raccordent les deux extrémités rentrantes du patin à la semelle plane(2) et comprennent une tige filetée permettant de régler la distance entre la semelle (2) et le patin (4).

6. Dispositif selon l'une quelconque des revendications précédentes, **caractérisé en ce que** le patin (4) est réalisé en un matériau métallique.

7. Dispositif selon la revendication 6, **caractérisé en ce que** le matériau métallique est choisi parmi l'aluminium, le titane est leurs alliages.

8. Dispositif selon l'une quelconque des revendications 1 à 5, **caractérisé en ce que** le patin (4) est réalisé en un matériau plastique.

9. Dispositif selon l'une quelconque des revendications précédentes, **caractérisé en ce que** la surface inférieure du patin est recouverte au moins partiellement par un matériau élastique (14).

10. Dispositif selon la revendication 9, **caractérisé en ce que** ce matériau élastique (14) est antidérapant.

11. Dispositif selon l'une quelconque des revendications précédentes, **caractérisé en ce que** le bord distal (24) du patin (4) présente une extension vers l'extérieur du pied de façon à prévenir une éventuelle torsion de la cheville lors de la marche.

12. Dispositif selon l'une quelconque des revendications précédentes, **caractérisé en ce que** la courbure du patin 4 est calculée par un programme informatique de simulation prenant en compte des caractéristiques anthropométriques du patient.

13. Procédé de fabrication d'un dispositif suivant l'une quelconque des revendications 1 à 12 **caractérisé en ce qu'il** comprend les opérations suivantes :
- Disposer d'un ordinateur ;
- Implémenter dans cet ordinateur un programme de simulation de la marche humaine dans lequel les dimensions anthropométriques d'un individu fictif affecté de LLI sont utilisées comme variables ;
- Implémenter dans ce programme la possibilité d'intégrer en outre la présence d'un dispositif de correction doté d'un patin (4), monté sur une des jambes ;
- Stocker dans un sous-programme des formes et des dimensions variables pour le patin de ce dispositif ;
- Prendre les mensurations anthropométriques d'un patient ;
- Entrer ces mensurations comme variables dans le programme de simulation de l'ordinateur ;
- Ajouter à ces variables la présence d'un dispositif de correction avec patin de forme arbitraire ;
- Vérifier la correction de la marche ainsi obtenue ;
- Faire varier la forme et les dimensions du patin ;
- Déterminer formes et dimensions du patin procurant la correction optimale ;
- Confectionner un patin (4) correspondant à cette forme optimale ;
- Fixer ce patin sur une semelle (2)
- Solidariser cette semelle à la semelle d'une chaussure (10).

14. Procédé de fabrication d'un dispositif suivant la revendication 13 **caractérisé en ce qu'il** comprend en outre l'opération suivante :
- Appliquer sur au moins une partie de la face inférieure du patin (4) une couche de matériau élastique (14).
